# EUROPÄISCHE PATENTSCHRIFT

(11) **EP 1 072 240 B1**
(45) Veröffentlichungstag und Bekanntmachung des Hinweises auf die Patenterteilung: **09.01.2002**
(21) Anmeldenummer: 00110308.4
(22) Anmeldetag: 13.05.2000
(51) Int. Cl.: A61F 2/60

(54) **Hüftgelenk für ein Kunstbein**
Hip joint for an artificial leg
Articulation de hanche pour une jambe artificielle

(30) Priorität: 27.07.1999 DE 19935203
(43) Veröffentlichungstag der Anmeldung: 31.01.2001
(73) Patentinhaber: Otto Bock HealthCare GmbH, 37115 Duderstadt (DE)
(72) Erfinder: Boiten, Herman, 3818 WG Amersfoort (NL); Wagner, Helmut, 37115 Duderstadt (DE)
(74) Vertreter: Gramm, Werner, Prof. Dipl.-Ing.

(56) Entgegenhaltungen:
- EP-A- 0 093 867
- EP-A- 0 285 328
- DE-C- 311 462

## Beschreibung

Die Erfindung betrifft ein Hüftgelenk für ein Kunstbein wobei die Oberseite des Hüftgelenkes Anschlussmittel zur Befestigung an einem Prothesenkorb aufweist, die Unterseite des Hüftgelenkes zur lösbaren Befestigung am Kunstbein ausgebildet ist, und ein Streckanschlag die Ausgangsposition des Hüftgelenkes in Streckstellung definiert.

Eine derartige Ausführungsform läßt sich der EP 0 093 867 B1 entnehmen. Bei diesem vorbekannten Hüftgelenk ist ein erstes, mit einem Anschlussmittel verschraubtes Gelenkteil über ein Drehgelenk mit einem zweiten, zur Verbindung mit dem Kunstbein vorgesehenen Gelenkteil verbunden, wobei die Drehbewegung des zweiten Gelenkteils durch Anschläge für die stehende und die sitzende Stellung des Kunstbeins beschränkt ist. Das Hüftgelenk ist einachsig ausgebildet und weist dementsprechend nur einen Freiheitsgrad auf, wobei das zweite Gelenkteil bzw. das mit ihm verbundene Kunstbein in nur einer Ebene verschwenkbar ist. Demgegenüber erfolgt die sehr viel kompliziertere Bewegung eines natürlichen Hüftgelenkes in drei Ebenen.

Eine mit dem eingangs beschriebenen Hüftgelenk vergleichbare Ausführungsform zeigt auch die EP 0 285 328 B1.

Der Erfindung liegt die Aufgabe zugrunde, ein Hüftgelenk der eingangs genannten Bauart zu entwickeln, das dem natürlichen Bewegungsablauf mehr angepasst ist und dadurch eine möglichst komfortable Gangart ermöglicht.

Diese Aufgabe wird bei einem Hüftgelenk der eingangs genannten Art erfindungsgemäß durch die im kennzeichnenden Teil des Anspruchs 1 angegebenen Merkmale gelöst.

Zur Beeinflussung der Gelenkkinematik ist es zweckmäßig, wenn die Länge des Frontlenkers bzw. der Abstand zwischen der ersten und der dritten Gelenkverbindung in Anpassung an den Prothesenträger justierbar ist. Durch eine Voreinstellung einer etwas größeren Länge des Frontlenkers werden Exorotation und Abduktion verändert. In einer abgewandelten Ausführungsform kann auch vorgesehen werden, dass der Frontlenker in Zugrichtung elastisch ausgebildet ist.

Ausgehend von der Streckstellung (0°-Position) ist eine Bewegung des Beines nach hinten eine Überstreckung (Hyperextension). Die während der Hyperextension und Beugung auftretende Rotation in der transversalen und frontalen Ebene nähert sich der natürlichen Hüftbewegung. Dabei wird eine Belastung des Kunstbeines durch Torsionsmomente verringert. Durch den dadurch erreichten leichteren Gang ist der Energieverbrauch beim Gehen geringer. Zudem läßt das erfindungsgemäß gestaltete Hüftgelenk eine komfortable Sitzposition zu.

Der Streckanschlag kann hart oder aber auch federelastisch ausgebildet sein. Dabei läßt sich die Streckstellung durch die Länge einer den Streckanschlag bildenden Feder definieren. Soll der Streckanschlag eine Hyperextension zulassen, kann er zweckmäßig als Federspeicher ausgebildet sein. Die Beaufschlagung des gegebenenfalls vorgespannten Federspeichers beginnt dann bei Streckstellung (0°-Postion gesehen in der Sagittalebene) und nimmt zu bis zur maximalen Überstreckung; nach der Kniebeugung entspannt dann der Federspeicher, dessen Entspannung bei Erreichen der Streckstellung (0° Flexion) endet. Bei Überstreckung wird somit in dem als Federspeicher ausgebildeten Streckanschlag Energie gespeichert, die am Anfang der Schwungphase wieder abgegeben wird und bei Erreichen der Streckstellung (0°-Position) aufgebraucht ist.

Weitere Merkmale der Erfindung sind Gegenstand der Unteransprüche und werden in Verbindung mit weiteren Vorteilen der Erfindung anhand von Ausführungsbeispielen näher erläutert.

In der Zeichnung ist eine als Beispiel dienende Ausführungsform der Erfindung dargestellt. Es zeigen
- Figur 1: in schematischer Darstellung in Frontansicht ein linkes, über ein Hüftgelenk mit einem Prothesenkorb verbundenes Kunstbein;
- Figur 2: in Streckstellung (0°-Position) ein linkes Hüftgelenk in Sagittal-Ansicht;

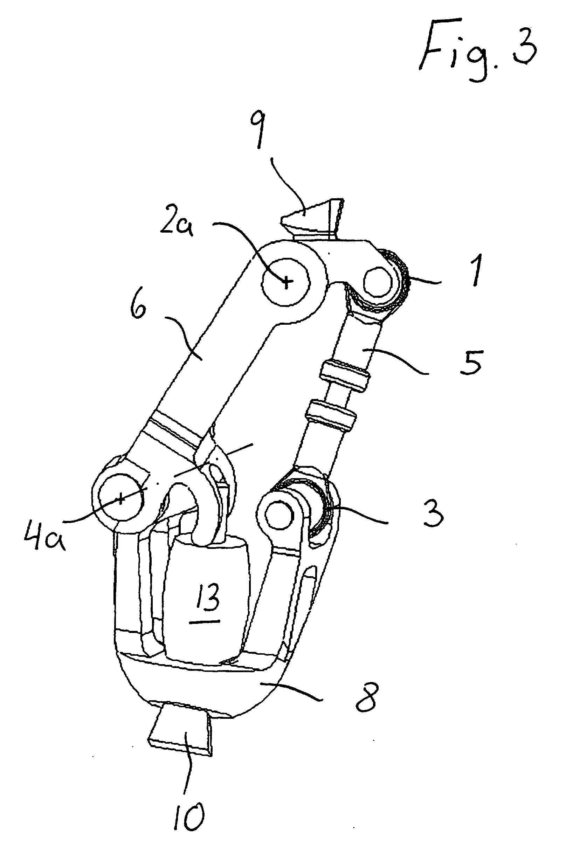

- Figur 3: in Sagittal-Ansicht das Hüftgelenk gemäß Figur 2 in 10°-Überstreckung;

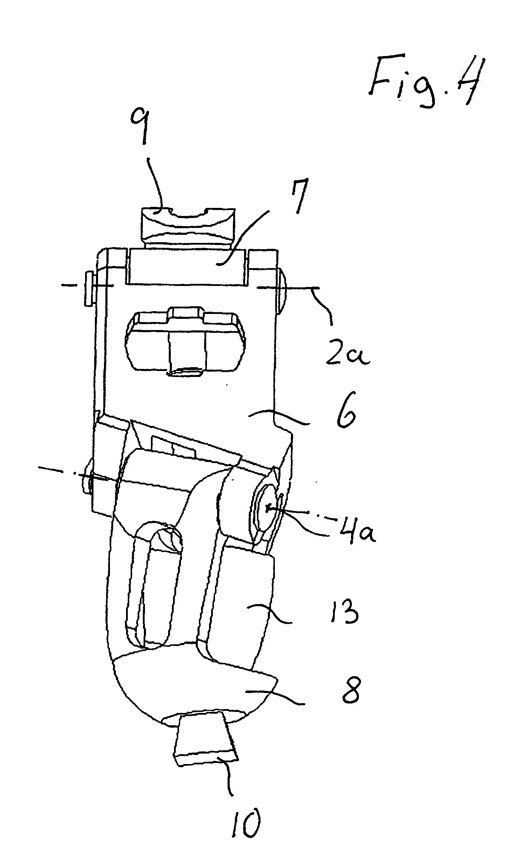

- Figur 4: die Darstellung gemäß Figur 3 in Rückansicht;

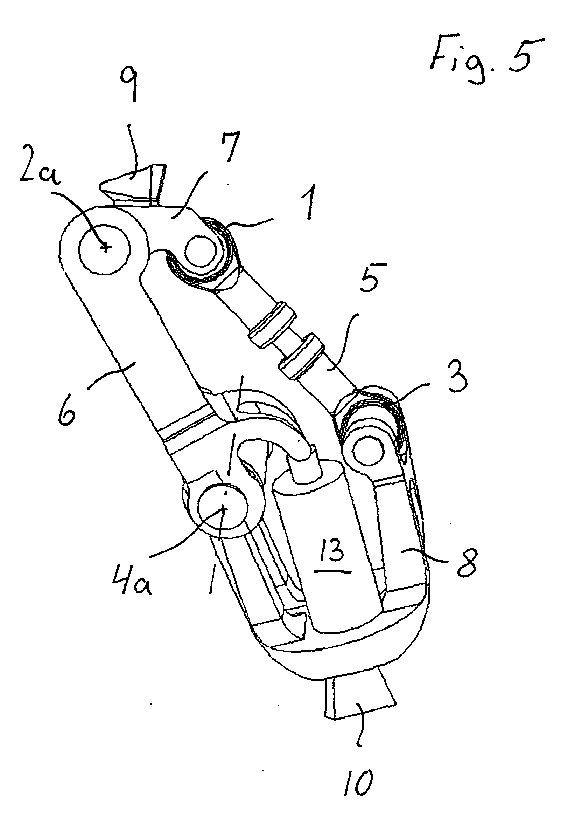

- Figur 5: in Sagittal-Ansicht das Hüftgelenk gemäß Figur 2 in 20°-Beugung;

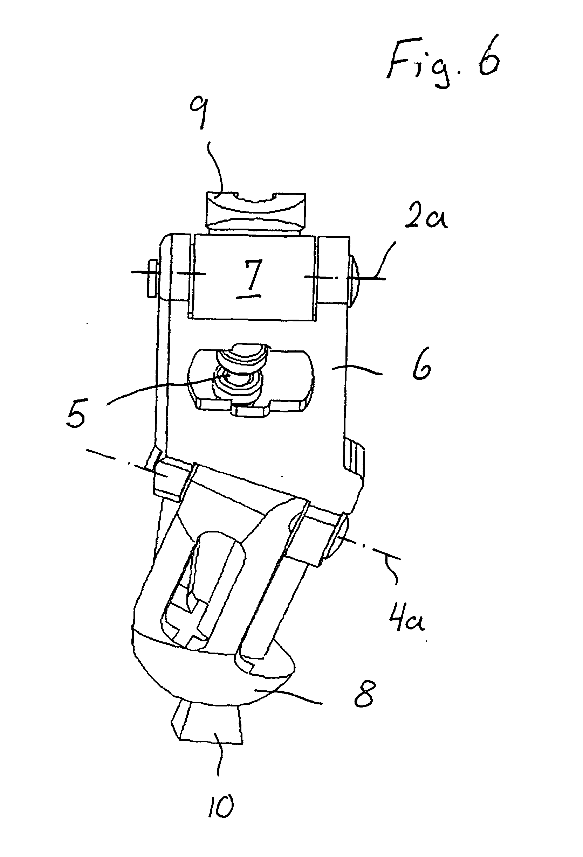

- Figur 6: die Darstellung gemäß Figur 5 in Rückansicht;

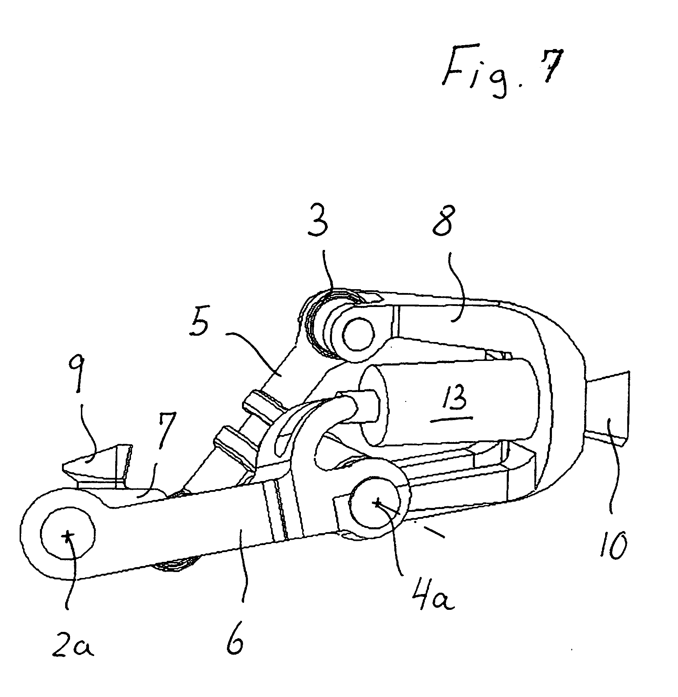

- Figur 7: in Sagittal-Ansicht das Hüftgelenk gemäß Figur 2 in 90°-Beugung und

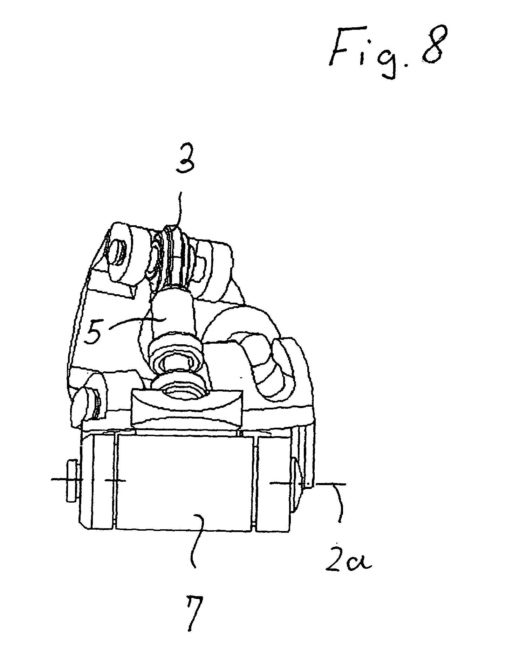

- Figur 8: die Darstellung gemäß Figur 7 in Rückansicht.

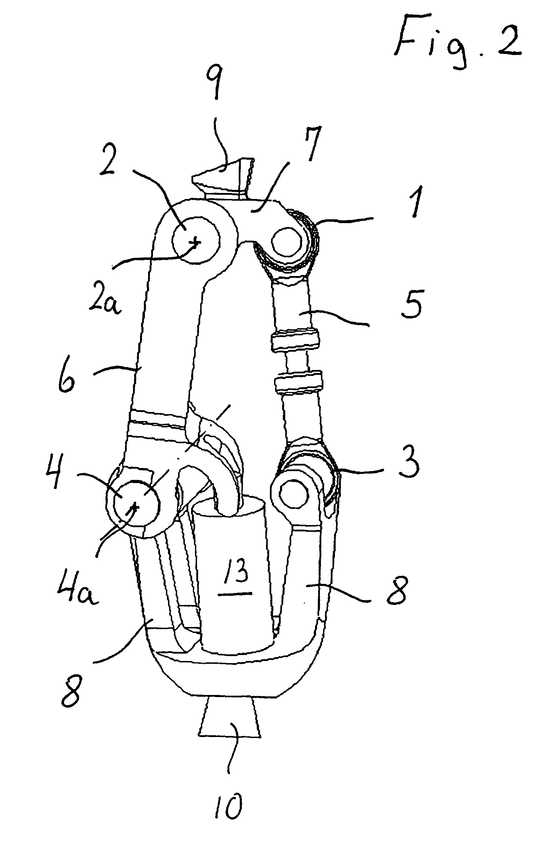

Das in Figur 2 dargestellte Hüftgelenk weist vier Gelenkverbindungen 1, 2, 3, 4 und insgesamt einen Freiheitsgrad auf. Ein Frontlenker 5 und ein Rücklenker 6 sind mit ihrem jeweils oberen Ende über die erste bzw. zweite Gelenkverbindung 1, 2 an einem vorderen bzw. hinteren Abschnitt eines oberen Querlenkers 7 und mit ihrem jeweils unteren Ende über die dritte bzw. vierte Gelenkverbindung 3, 4 an einem vorderen bzw. hinteren Abschnitt eines unteren Querlenkers 8 angelenkt.

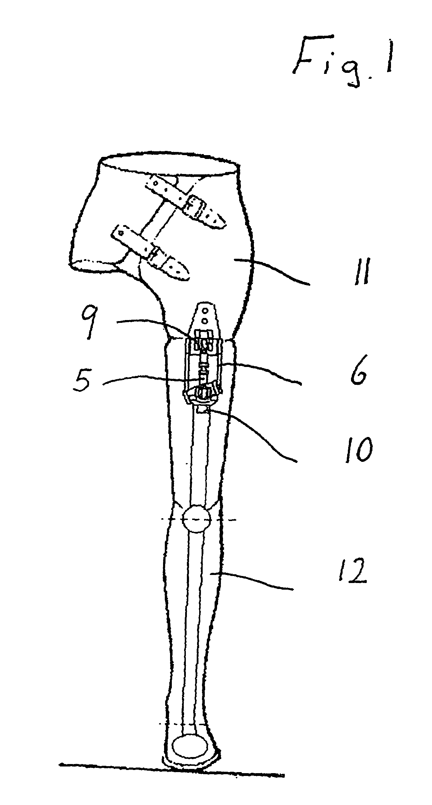

Der obere Querlenker 7 ist mit Anschlußmitteln 9 zur Befestigung an einem Prothesenkorb 11 und der untere Querlenker 8 mit einem Adapter 10 zur lösbaren Befestigung an einem Kunstbein 12 versehen (siehe auch Figur 1).

Die erste und die dritte Gelenkverbindung 1, 3 weisen zusammen mindestens fünf Freiheitsgrade auf und können entweder durch je ein Kugelgelenk, oder aber durch je ein Kardangelenk gebildet sein, wobei im letzteren Falle eine zusätzliche Drehverbindung erforderlich ist, die vorzugsweise durch einen um seine Längsachse drehbaren Frontlenker 5 gebildet wird, der die beiden Kardangelenke miteinander verbindet. Auch eine Kombination von Kugelgelenk und Kardangelenk ist möglich.

Die zweite Gelenkverbindung 2 wird durch eine Gelenkachse gebildet, die mit ihrer Drehachse 2a angenähert senkrecht auf der Sagittalebene steht und einen Freiheitsgrad aufweist.

Die vierte Gelenkverbindung 4 weist eine mediolateral sowie von vorn nach hinten geneigte Drehachse 4a mit einem Freiheitsgrad auf, so daß eine Verschwenkung des unteren Querlenkers 8 um diese Drehachse 4a im Uhrzeigersinn eine Exoverschwenkung des angeschlossenen Kunstbeines 12 mit nach außen gerichteter Abduktion bewirkt, während die Verschwenkung entgegen dem Uhrzeigersinn zu einer Endoverschwenkung und Adduktion führt. Frontal gesehen liegt also das laterale Ende der Drehachse 4a höher als das mediale Ende, wobei frontal gesehen diese Neigung etwa 17,5° beträgt. Diese Neigung hat hauptsächlich Einfluß auf die Exo- und Endorotation. Von oben gesehen liegt das laterale Ende der Drehachse 4a weiter nach frontal, das mediale Ende also weiter nach posterior, wobei diese Schiefstellung vorzugsweise etwa 17° beträgt und hauptsächlich Einfluß hat auf die Abduktion bzw. Adduktion. Beim normalen Gehen führt diese Ausführungsform zu einer maximalen Exorotation des Kunstbeines von etwa 10° und zu einer maximalen Abduktion von etwa 5°. Dabei führt das Hüftgelenk während der Hüftstreckung zu einer abnehmenden Abduktion, wodurch der Fuß dichter unter den Körperschwerpunkt kommt.

Bei der Darstellung gemäß Figur 2 bewirkt eine Verschwenkung des unteren Querlenkers 8 um die Drehachse 4a im Uhrzeigersinn eine Flexion des Kunstbeines 12. Ist nach einer bestimmten Flexion eine weitere Verschwenkung im Uhrzeigersinn nicht mehr möglich, kehrt sich die Richtung der Verschwenkung um, findet also bei weiterer Flexion entgegen dem Uhrzeigersinn statt. Dadurch verringern sich Exoverschwenkung und Abduktion, wodurch eine normale Sitzposition erreicht wird. Anders ausgedrückt führt eine Verschwenkung des Rücklenkers 6 um die Drehachse 4a entgegen dem Uhrzeigersinn zu einer Vergrößerung des Winkels zwischen Rücklenker 6 und unterem Querlenker 8. Dieser Winkel erreicht ein Maximum und verringert sich anschließend wieder. Nach weiterem Anstieg des Flexionswinkels des gesamten Hüftgelenkes erreicht der zwischen Rücklenker 6 und unterem Querlenker 8 eingeschlossene Winkel wieder annähernd seinen Ausgangswert, was etwa der Sitzposition entspricht, in der das Kunstbein eine geringe Abduktion und Exorotation aufweist.

Das in Figur 2 dargestellte Hüftgelenk läßt ferner einen Streckanschlag 13 erkennen, der die Ausgangsposition des Hüftgelenkes bei 0°, also die Streckstellung definiert.

zusätzlich kann noch ein die Schwungphase begrenzender Anschlag vorgesehen sein. Das Hüftgelenk kann ferner mit einer Dämpfung ausgestattet sein.

## Patentansprüche

1. Hüftgelenk für ein Kunstbein (12), wobei die Oberseite des Hüftgelenkes Anschlussmittel (9) zur Befestigung an einem Prothesenkorb (11) aufweist, die Unterseite des Hüftgelenkes zur lösbaren Befestigung am Kunstbein ausgebildet ist, und ein Streckanschlag (13) die Ausgangsposition des Hüftgelenkes in Streckstellung definiert,
**gekennzeichnet durch** folgende Merkmale:
a) ein Frontlenker (5) und ein Rücklenker (6) sind mit ihrem jeweils oberen Abschnitt über eine erste (1) bzw. zweite (2) Gelenkverbindung an einem vorderen bzw. hinteren Abschnitt eines oberen Querlenkers (7) und mit ihrem jeweils unteren Abschnitt über eine dritte (3) bzw. vierte (4) Gelenkverbindung an einem vorderen bzw. hinteren Abschnitt eines unteren Querlenkers (8) angelenkt;
b) der obere Querlenker (7) ist mit den Anschlussmitteln (9) zur Befestigung an einem Prothesenkorb (11) und der untere Querlenker (8) mit einem Adapter (10) zur lösbaren Befestigung an dem Kunstbein (12) versehen;
c) die erste und die dritte Gelenkverbindung (1, 3) weisen zusammen zumindest fünf Freiheitsgrade auf;
d) die zweite Gelenkverbindung (2) ist eine Gelenkachse, die mit ihrer Drehachse (2a) angenähert senkrecht auf der Sagittalebene steht und einen Freiheitsgrad aufweist;
e) die vierte Gelenkverbindung (4) weist eine mediolateral sowie von vorn nach hinten geneigte Drehachse (4a) mit einem Freiheitsgrad auf, so daß eine Verschwenkung um die Drehachse (2a) der zweiten Gelenkverbindung (2) eine Exo-/Endoverschwenkung und eine Ab-/Adduktion des unteren Querlenkers (8) bewirkt.

2. Hüftgelenk nach Anspruch 1, **dadurch gekennzeichnet, daß** die erste und dritte Gelenkverbindung (1, 3) durch je ein Kugelgelenk gebildet sind.

3. Hüftgelenk nach Anspruch 1, **dadurch gekennzeichnet, daß** die erste und dritte Gelenkverbindung (1, 3) durch je ein Kardangelenk gebildet sind, die durch den Frontlenker (5) miteinander verbunden sind, der ums eine Längsachse drehbar ausgebildet ist.

4. Hüftgelenk nach Anspruch 1, 2 oder 3, **dadurch gekennzeichnet, daß** die Länge des Frontlenkers (5) bzw. der Abstand zwischen der ersten und der dritten Gelenkverbindung (1, 3) in Anpassung an den Prothesenträger justierbar ist.

5. Hüftgelenk nach Anspruch 1, 2 oder 3, **dadurch gekennzeichnet, daß** der Frontlenker (5) in Zugrichtung elastisch ausgebildet ist.

6. Hüftgelenk nach einem der vorhergehenden Ansprüche, **dadurch gekennzeichnet, daß** der Streckanschlag (13) federelastisch ausgebildet ist.

7. Hüftgelenk nach Anspruch 6, **dadurch gekennzeichnet, daß** die Streckstellung durch die Länge einer den Streckanschlag (13) bildenden Feder definiert ist.

8. Hüftgelenk nach Anspruch 7, **dadurch gekennzeichnet, daß** der Streckanschlag (13) als Federspeicher ausgebildet ist.

9. Hüftgelenk nach einem der vorhergehenden Ansprüche, **dadurch gekennzeichnet, daß** die Drehachse (4a) der vierten Gelenkverbindung (4) frontal gesehen eine Neigung von etwa 17° - 18° aufweist.

10. Hüftgelenk nach einem der vorhergehenden Ansprüche, **dadurch gekennzeichnet, daß** die Drehachse (4a) der vierten Gelenkverbindung (4) von oben gesehen eine Schiefstellung von etwa 17° aufweist.

## Claims

1. Hip-joint for an artificial leg (12), wherein the upper side of the hip-joint has connection means (9) for fastening to a prosthesis cage (11), the underside of the hip-joint is formed to be detachably fastened to the artificial leg, and an extension stop (13) defines the initial position of the hip-joint when extended,
**characterised by** the following features:
a) a front link (5) and a rear link (6) are articulated by way of their respective upper portions via a first (1) and a second (2) articulation, respectively, to a front and a rear portion, respectively, of an upper transverse link (7) and by way of their respective lower portions via a third (3) and a fourth (4) articulation to a front and a rear portion, respectively, of a lower transverse link (8);
b) the upper transverse link (7) is provided with the connection means (9) for fastening to a prosthesis cage (11), and the lower transverse link (8) is provided with an adapter (10) for detachable fastening to the artificial leg (12);
c) the first and the third articulation (1, 3) jointly have at least five degrees of freedom;
d) the second articulation (2) is a joint pin which is positioned with its rotational axis (2a) approximately perpendicularly to the sagittal plane and has one degree of freedom;
e) the fourth articulation (4) has a mediolateral rotational axis (4a) which is inclined from the front to the rear with one degree of freedom, so that a swinging movement about the rotational axis (2a) of the second articulation (2) gives rise to an exo-/endo-swinging movement and an abduction/adduction of the lower transverse link (8).

2. Hip-joint according to Claim 1, **characterised in that** the first and the third articulation (1, 3) are each formed by a respective ball-and-socket joint.

3. Hip-joint according to Claim 1, **characterised in that** the first and the third articulation (1, 3) are each formed by a respective universal joint, these being connected together by the front link (5), which is formed so as to be rotatable about a longitudinal axis.

4. Hip-joint according to Claim 1, 2 or 3, **characterised in that** the length of the front link (5) or the spacing between the first and the third articulation (1, 3) can be adjusted so as to adapt to the person wearing the prosthesis.

5. Hip-joint according to Claim 1, 2 or 3, **characterised in that** the front link (5) is elastic in the direction of pull.

6. Hip-joint according to any one of the preceding Claims, **characterised in that** the extension stop (13) is resilient.

7. Hip-joint according to Claim 6, **characterised in that** the extended position is defined by the length of a spring forming the extension stop (13).

8. Hip-joint according to Claim 7, **characterised in that** the extension stop (13) is formed as a storage spring.

9. Hip-joint according to any one of the preceding Claims, **characterised in that** the rotational axis (4a) of the fourth articulation (4) is inclined by approximately 17° - 18° when viewed from the front.

10. Hip-joint according to any one of the preceding Claims, **characterised in that** the rotational axis (4a) of the fourth articulation (4) is at a slant of approximately 17° when viewed from above.

## Revendications

1. Articulation de la hanche pour jambe artificielle (12), dans laquelle le côté supérieur de l'articulation de la hanche présente un moyen de raccordement (9) pour fixation à une coque prothétique (11), le côté inférieur de l'articulation de la hanche est agencé pour fixation démontable à la jambe artificielle, et une butée d'extension (13) définit la position de départ de l'articulation de la hanche en position d'extension,
**caractérisée par** les particularités suivantes :
a) une biellette antérieure (5) et une biellette postérieure (6) sont articulées chacune par leur partie supérieure, par l'intermédiaire d'une première (1) et respectivement d'une seconde (2) liaison articulée à une partie antérieure et respectivement postérieure d'une biellette transversale supérieure (7), et par leur partie inférieure respective, par l'intermédiaire d'une troisième (3) et respectivement d'une quatrième (4) liaison articulée à une partie antérieure et respectivement postérieure d'une biellette transversale inférieure (8) ;
b) la biellette transversale supérieure (7) est munie des moyens de raccordement (9) pour fixation à une coque prothétique (11) et la biellette transversale inférieure (8) est munie d'un adaptateur (10) pour fixation démontable à la jambe artificielle (12) ;
c) la première et la troisième liaison articulée (1,3) présentent ensemble au moins cinq degrés de liberté ;
d) la deuxième liaison articulée (2) est un axe d'articulation dont l'axe de pivotement (2a) s'étend sensiblement perpendiculairement au plan sagittal et présente un degré de liberté ;
e) la quatrième liaison articulée (4) présente un axe d'articulation (4a) incliné médio-latéralement ainsi que de l'avant vers l'arrière et comportant un degré de liberté, de sorte qu'un pivotement autour de l'axe d'articulation (2a) de la deuxième liaison articulée (2) produit un exopivotement/endopivotement et une abduction/adduction de la biellette transversale inférieure (8).

2. Articulation de la hanche selon la revendication 1, **caractérisée en ce que** la première et la troisième liaison articulée (1,3) sont réalisées chacune par une rotule.

3. Articulation de la hanche selon la revendication 1, **caractérisée en ce que** première et la troisième liaison articulée (1,3) sont constituées chacune par un joint de cardan, qui sont reliées l'un à l'autre par la biellette antérieure (5) qui est réalisée rotative autour de son axe longitudinal.

4. Articulation de la hanche selon la revendication 1, 2 ou 3, **caractérisée en ce que** la longueur de la biellette antérieure (5) et respectivement la distance entre la première et la troisième liaison articulée (1,3) est réglable pour être adaptée au porteur de prothèse.

5. Articulation de la hanche selon la revendication 1, 2 ou 3, **caractérisée en ce que** la biellette antérieure (5) est réalisée élastique en traction.

6. Articulation de la hanche selon l'une quelconque des revendications précédentes, **caractérisée en ce que** la butée d'extension (13) est réalisée élastique.

7. Articulation de la hanche selon la revendication 6, **caractérisée en ce que** la position d'extension est définie par la longueur d'un ressort constituant la butée d'extension (13).

8. Articulation de la hanche selon la revendication 7, **caractérisée en ce que** la butée d'extension (13) est réalisée sous la forme d'un accumulateur à ressort.

9. Articulation de la hanche selon l'une quelconque des revendications précédentes, **caractérisée en ce que** l'axe de pivotement (4a) de la quatrième liaison articulée (4) présente en vue frontale une inclinaison d'environ 17-18°.

10. Articulation de la hanche selon l'une quelconque des revendications précédentes, **caractérisée en ce que** l'axe de pivotement (4a) de la quatrième liaison articulée (4) présente en vue de dessus une obliquite d'environ 17°.
